Europäisches Patentamt

European Patent Office

Office européen des brevets

(11) Publication number: **0 452 505 A1**

(12) **EUROPEAN PATENT APPLICATION**
published in accordance with Art.
158(3) EPC

(21) Application number: 90916078.0

(22) Date of filing: 06.11.90

(86) International application number:
PCT/JP90/01439

(87) International publication number:
WO 91/06567 (16.05.91 91/11)

(51) Int. Cl.⁵: **C07K 13/00**, C12N 15/27,
C12N 1/21, C12P 21/02,
//A61K37/02,(C12N1/21,
C12R1:19),(C12P21/02,
C12R1:19)

(30) Priority: 07.11.89 JP 290730/89

(43) Date of publication of application:
23.10.91 Bulletin 91/43

(84) Designated Contracting States:
CH DE DK ES FR GB IT LI NL SE

(71) Applicant: OTSUKA PHARMACEUTICAL CO.,
LTD.
9, Kandatsukasacho 2-chome
Chiyoda-ku Tokyo 101(JP)

(72) Inventor: TAKAHASHI, Masayuki
79-7, Aza Nakanokoshi Kisuno, Otsucho
Naruto-shi Tokushima 772(JP)
Inventor: NAKAI, Satoru
67-4, Aza Minamikawamuko Hiroshima
Matsushigecho
Itano-gun Tokushima 771-02(JP)
Inventor: NISHIDA, Tsutomu
240-118, Oshiro Otsucho
Naruto-shi Tokushima 772(JP)
Inventor: TAKANO, Masaaki
77-6, Aza Inui Sumiyoshi
Aizumicho Itano-gun Tokushima 771-12(JP)

Inventor: SEKIGUCHI, Yasuyo Room 302 Dai-3
Aijitsu Haitsu
59-3, Shozui Seicho Aizumicho
Itano-gun Tokushima 771-12(JP)
Inventor: YAMANISHI, Kazuya Room 401 Dai-2
Kopo Asahina
4-30, Shinhamahoncho 4-chome
Tokushima-shi Tokushima 770(JP)
Inventor: OMOTO, Yasukazu
35-6, Aza Hachishimo Sasagino
Matsushigecho
Itano-gun Tokushima 771-02(JP)
Inventor: ICHIKAWA, Hiroyuki
11, Nakatourimachi 3-chome Tokushima-shi
Tokushima 770(JP)
Inventor: HIRAI, Yoshikatsu
5-49, Aza Ekogawa Shinkirai Kitajimacho
Itano-gun Tokushima 771-02(JP)

(74) Representative: Kolb, Helga, Dr. Dipl.-Chem. et
al
Hoffmann, Eitle & Partner Patentanwälte
Arabellastrasse 4
W-8000 München 81(DE)

(54) M-CSF DERIVATIVE, EXPRESSION VECTOR OF SAID DERIVATIVE, PRODUCT OF TRANSFORMATION BY SAID VECTOR, AND PRODUCTION OF THEM.

(57) The invention provides a biologically active recombinant human M-CSF derivative characterized by having a primary amino acid sequence wherein a C-terminus is present somewhere in the region ranging from 153-position Thr to 522-position Val while an N-terminus is present at 3-position Val or 4-position Ser and Met may optionally be added to the N-terminus of such an amino acid sequence in the mature human M-CSF amino acid sequence; an expression vector of said M-CSF derivative; a product of transformation caused by said vector;

and a method of producing the M-CSF derivative by culturing said product of transformation and taking up the M-CSF derivative thus expressed.

TECHNICAL FIELD

The present invention relates to human colony-stimulating factors (CSFs), in particular recombinant human M-CSF derivatives, which are useful as drugs, expression vectors for said derivatives, transformants carring said vectors and methods of producing these.

PRIOR ART

Generally, specific proliferation and differentiation factors are required for the proliferation and differentiation of hematopoietic cells. Various such factors participate in the differentiation and proliferation of hematopoietic cells into various mature blood cells, such as erythrocytes, granulocytes, macrophages, eosinophils, platelets and lymphocytes (Yasusada Miura, "Blood Stem Cells," Chugai Igaku Sha, 1983). Among these factors, CSFs are known as factors which stimulate the proliferation and differentiation of granulocyte precursor cells and macrophage precursor cells. Known as such CSFs are the G-type one (G- CSF) which is specific to the formation of granulocytes, the M-type one (M-CSF) which is specific to the formation of macrophages, and the GM-type one (GM-CSF) which promotes the formation of both granulocytes and macrophages. Furthermore, multi-CSF (IL-3) is also known as a CSF which acts on pluripotent stem cells.

While cancer chemotherapy and radiotherapy cause leukopenia as a common drawback, the above- mentioned CSFs, because of their biological activity, are thought to alleviate such leukopenia. Clinical researches on CSFs are under way from this viewpoint.

The CSFs are also known to have activity to promote the function of leukocytes [Lopez, A. F. et al., J. Immunol., 131, 2983 (1983), Handam, E. et al., ibid., 122, 1134 (1979) and Vadas, M. A. et al., ibid., 130, 795 (1983)] and are therefore found effective as drugs for preventing and curing various infectious diseases. · Furthermore, the CSFs are found effective in curing myelogenous leukemia because of their differentiation inducing activity [Metcalf, D. et al., Int. J. Cancer, 30, 773 (1982)].

The activity of CSFs is found, for example, in the cultures of fetal cells, spleen cells, etc., in human urine and in the culture media containing various incubated cell lines, and active fractions thereof are separated off and used. However, the CSF of any origin contains large quantities of analogous extraneous substances or the like derived from the starting material and is in itself low in concentration, so that these problems must be solved for preparing the CSF. In respect of homogeneity, yield, ease of operation, etc., therefore, any means capable of commercially and constantly producing CSFs as drugs is not yet available as yet.

We have already established "ARG-ON", a cell line derived from human leukemic T cells, which is capable of producing a large quantity of CSF constitutively in a homogeneous state, and filed a patent application claiming an invention relating to this cell line (Unexamined Japanese Patent Publication SHO 59- 169489). As a result of further experiments on the purification of the CSF produced by ARG-ON, we have also developed a process for preparing the CSF easily in a pure form and in a high yield. We have further clarified the structural and biochemical characteristics of the thus-obtained CSF and completed an invention relating to the CSF as a substance (Unexamined Japanese Patent Publication SHO 62-169799).

Furthermore, for producing the above-mentioned CSF by using genetic engineering techniques, we have developed a gene coding for said CSF, a vector containing said gene, and a COS cell expression system, an Escherichia coli secretory expression system and a CHO cell expression system where, in each expression system, the relevant cells are transformed with said vector, and filed an application for patent on the invention concerning these (Unexamined Japanese Patent Publication HEI 1-104176).

It is a primary object of the present invention to provide a technology of producing biologically active human M-CSF derivatives and further provide novel human M-CSF derivatives obtainable by said technology and, in particular, more useful as drugs.

DISCLOSURE OF THE INVENTION

The present invention provides biologically active recombinant human M-CSF derivatives characterized by having a primary amino acid sequence derived from the primary amino acid sequence defined below by formula (1) by putting an end (C terminus) at a position within the range of position 151 (Thr) to position 520 (Val), optionally with addition of Met to the N terminus of said sequence and/or deletion of the N-terminal Val therefrom, in particular biologically active recombinant human M-CSF derivatives having a primary amino acid sequence comprising a segment of the primary amino acid sequence defined below by formula (1) which segment extends from position 1 (Val) to position 182 (Ala), from position 1 (Val) to position 212

3

(Pro), from position 1 (Val) to position 256 (Gly), from position 1 (Val) to position 300 (Gln), from position 1 (Val) to position 332 (Ala) or from position 1 (Val) to position 520 (Val), or from position 2 (Ser) to position 151 (Thr), from position 2 (Ser) to position 182 (Ala), from position 2 (Ser) to position 212 (Pro), from position 2 (Ser) to position 256 (Gly), from position 2 (Ser) to position 300 (Gln) or from position 2 (Ser) to position 332 (Ala), optionally with addition of Met to the N terminus thereof.

Formula (I):

Val-Ser-Glu-Tyr-Cys-Ser-His-Met-Ile-Gly-Ser-Gly-

His-Leu-Gln-Ser-Leu-Gln-Arg-Leu-Ile-Asp-Ser-Gln-

Met-Glu-Thr-Ser-Cys-Gln-Ile-Thr-Phe-Glu-Phe-Val-

Asp-Gln-Glu-Gln-Leu-Lys-Asp-Pro-Val-Cys-Tyr-Leu-

Lys-Lys-Ala-Phe-Leu-Leu-Val-Gln-Asp-Ile-Met-Glu-

Asp-Thr-Met-Arg-Phe-Arg-Asp-Asn-Thr-Pro-Asn-Ala-

Ile-Ala-Ile-Val-Gln-Leu-Gln-Glu-Leu-Ser-Leu-Arg-

Leu-Lys-Ser-Cys-Phe-Thr-Lys-Asp-Tyr-Glu-Glu-His-

Asp-Lys-Ala-Cys-Val-Arg-Thr-Phe-Tyr-Glu-Thr-Pro-

Leu-Gln-Leu-Leu-Glu-Lys-Val-Lys-Asn-Val-Phe-Asn-

Glu-Thr-Lys-Asn-Leu-Leu-Asp-Lys-Asp-Trp-Asn-Ile-

Phe-Ser-Lys-Asn-Cys-Asn-Asn-Ser-Phe-Ala-Glu-Cys-

Ser-Ser-Gln-Asp-Val-Val-Thr-Lys-Pro-Asp-Cys-Asn-

4

Cys-Leu-Tyr-Pro-Lys-Ala-Ile-Pro-Ser-Ser-Asp-Pro-
Ala-Ser-Val-Ser-Pro-His-Gln-Pro-Leu-Ala-Pro-Ser-
Met-Ala-Pro-Val-Ala-Gly-Leu-Thr-Trp-Glu-Asp-Ser-
Glu-Gly-Thr-Glu-Gly-Ser-Ser-Leu-Leu-Pro-Gly-Glu-
Gln-Pro-Leu-His-Thr-Val-Asp-Pro-Gly-Ser-Ala-Lys-
Gln-Arg-Pro-Pro-Arg-Ser-Thr-Cys-Gln-Ser-Phe-Glu-
Pro-Pro-Glu-Thr-Pro-Val-Val-Lys-Asp-Ser-Thr-Ile-
Gly-Gly-Ser-Pro-Gln-Pro-Arg-Pro-Ser-Val-Gly-Ala-
Phe-Asn-Pro-Gly-Met-Glu-Asp-Ile-Leu-Asp-Ser-Ala-
Met-Gly-Thr-Asn-Trp-Val-Pro-Glu-Glu-Ala-Ser-Gly-
Glu-Ala-Ser-Glu-Ile-Pro-Val-Pro-Gln-Gly-Thr-Glu-
Leu-Ser-Pro-Ser-Arg-Pro-Gly-Gly-Gly-Ser-Met-Gln-
Thr-Glu-Pro-Ala-Arg-Pro-Ser-Asn-Phe-Leu-Ser-Ala-
Ser-Ser-Pro-Leu-Pro-Ala-Ser-Ala-Lys-Gly-Gln-Gln-
Pro-Ala-Asp-Val-Thr-Gly-Thr-Ala-Leu-Pro-Arg-Val-
Gly-Pro-Val-Arg-Pro-Thr-Gly-Gln-Asp-Trp-Asn-His-
Thr-Pro-Gln-Lys-Thr-Asp-His-Pro-ser-Ala-Leu-Leu-
Arg-Asp-Pro-Pro-Glu-Pro-Gly-Ser-Pro-Arg-Ile-Ser-
Ser-Pro-Arg-Pro-Gln-Gly-Leu-Ser-Asn-Pro-Ser-Thr-
Leu-Ser-Ala-Gln-Pro-Gln-Leu-Ser-Arg-Ser-His-Ser-
Ser-Gly-Ser-Val-Leu-Pro-Leu-Gly-Glu-Leu-Glu-Gly-
Arg-Arg-Ser-Thr-Arg-Asp-Arg-Arg-Ser-Pro-Ala-Glu-
Pro-Glu-Gly-Gly-Pro-Ala-Ser-Glu-Gly-Ala-Ala-Arg-
Pro-Leu-Pro-Arg-Phe-Asn-Ser-Val-Pro-Leu-Thr-Asp-

```
Thr-Gly-His-Glu-Arg-Gln-Ser-Glu-Gly-Ser-Ser-Ser-

Pro-Gln-Leu-Gln-Glu-Ser-Val-Phe-His-Leu-Leu-Val-

Pro-Ser-Val-Ile-Leu-Val-Leu-Leu-Ala-Val-Gly-Gly-

Leu-Leu-Phe-Tyr-Arg-Trp-Arg-Arg-Arg-Ser-His-Gln-

Glu-Pro-Gln-Arg-Ala-Asp-Ser-Pro-Leu-Glu-Gln-Pro-

Glu-Gly-Ser-Pro-Leu-Thr-Gln-Asp-Asp-Arg-Gln-Val-

Glu-Leu-Pro-Val
```

Peptides and amino acids are herein referred to by symbols according to the amino acid nomenclature adopted by IUPAC or by symbols convenientlY used in the art. DNA base sequences and nucleic acids are also expressed similarly.

The human M-CSF derivatives of the present invention have M-CSF activity and, because of their biological activity, are useful in the medicinal field, for example as drugs for preventing and curing diseases or pathologic states accompanied by leukopenia, as auxiliary agents for bone marrow transplantation, as drugs for preventing and curing various infectious diseases and further as anticancer agents, etc., as mentioned hereinbefore. Furthermore, the human M-CSF derivatives of the present invention are recombinant-type M-CSF derivatives prepared by using genetic engineering techniques with E. coli as the host and, owing to their homogeneity, are useful particularly in the above fields.

In addition, among the derivatives of the present invention some are comparable in blood half-life to the M-CSF [shown later herein in Reference Example 1] expressed in animal cells and having a molecular weight of about 85,000. Thus, these M-CSF derivatives expressed in E. coli and having M-CSF activity show a long half-life in vivo and can be produced easily at low cost as homogeneous substances as compared with the M-CSF expressed in animal cells.

Each human M-CSF derivative of the present invention is prepared utilizing a gene coding for the same, i.e. by preparing a recombinant DNA for the expression of said gene in host cells, introducing the DNA into host cells for transformation thereof and cultivating the resulting transformant.

The gene for the production of a human M-CSF derivative of the present invention can be prepared starting with mRNA isolated from various human cells having ability to produce M-CSF, more specifically and advantageously from the above-mentioned AGR-ON [human leukemic T cell-derived cultured cell line having the characteristics described in Unexamined Japanese Patent Publication SHO 59-169489; deposited in the American Type Culture Collection (ATCC) under the ATCC deposition No. CRL-8199]. The extraction procedure for the isolation of mRNA from said AGR-ON can be carried out, for example by the guanidinium/hot phenol method [T. Maniatis, E. F. Fritsch and J. Sambrook, Molecular Cloning, pp. 194-195 (Cold Spring Harbor Laboratory), 1982], the guanidinium/cesium chloride method [T. Maniatis, E. F. Fritsch and J. Sambrook, Molecular Cloning, p. 196 (Cold Spring Harbor Laboratory), 1982] or the like. The conversion of the purified mRNA to cDNA, namely the synthesis of the desired gene, can be realized, for example by the Okayama-Berg method [H. Okayama and P. Berg, Molecular and Cellular Biology, vol. 3, p. 280 (1983)], the Gubler-Hoffman method [V. Gubler and B. J. Hoffman, Gene, vol. 25, pp. 263-269 (1983)] or the like. The transformation by introducing the thus-obtained DNA into host cells and the selection of a desired strain carrying the M-CSF cDNA from among the transformants can be performed by conventional methods. The above-cited Unexamined Japanese Patent Publication HEI 1-104176 describes these procedures or methods in detail.

The M-CSF gene described in said patent specification also can be advantageously employed as the M-CSF gene to be used in the practice of the present invention.

The genes mentioned above can also be prepared in the manner of chemical synthesis of nucleotides using a conventional method such as the phosphite triester method [Nature, 310, 105 (1984)]. In any of the methods, various procedures or steps, such as partial DNA synthesis by chemical means, enzyme treatments for DNA chain cleavage, deletion, addition and ligation, DNA isolation, purification and replication, screening, etc., can be performed in the conventional manner. For example, the above-mentioned DNA isolation and purification can be carried out by agarose gel electrophoresis, etc., and partial modification of a codon in the nucleic acid sequence can be conducted by site-specific mutagenesis [Proc. Natl. Acad. Sci., 81, 5662-5666 (1984)], etc. The codon to be selected for a desired amino acid is not limited specifically but can be determined in a usual manner in view of the codon usage for the host cell to be utilized, etc. The DNA sequence of the desired gene to be used in the above methods can be determined and confirmed, for example, by the Maxam-Gilbert chemical modification method [Methods in Enzymolozy, 65, 499-560 (1980)] or by the dideoxynucleotide chain termination method using M13 phage [Messing, J. and Vieira, J., Gene, 19, 269-276 (1982)].

The human M-CSF derivatives of the present invention can be produced easily in large quantities by recombinant DNA techniques using the genes obtained in the above manner. While the use of the above-mentioned specific genes is essential, this process can be conducted basically by general genetic engineering techniques [cf. Molecular Cloning, T. Maniatis et al., Cold Spring Harbor Laboratory (1982), etc.].

More specifically, a recombinant DNA which can express a gene of the invention in host cells, introducing the DNA into the host cell for transformation thereof and cultivating the transformant.

Useful host cells can be either eukaryotic or prokaryotic cells. Generally used as the prokaryotic hosts are E. coli and Bacillus subtilis. In the practice of the invention, a plasmid vector capable of replication in such a host, for instance, is employed and, for enabling expression of an M-CSF gene in said plasmid vector, an expression vector is prepared by inserting said gene into said plasmid vector and providing a promoter and an SD (Shine-Dalgarno) sequence and further an initiation codon (e.g. ATG) required for the initiation of protein synthesis upstream from said gene. Widely used as host E. coli is the strain K12. pBR322 is generally used as the vector. However, these are not limitative but various known strains and vectors can be used. Useful as the promoter are, for example, the tryptophan (trp) promoter, lpp promoter, lac promoter, $P_L$ promoter, etc. and, in any case, the desired gene can be expressed.

As a preferred process for producing the human M-CSF derivatives of the present invention using the genes mentioned above, there may be mentioned, by way of example, a process which uses a prokaryotic host, such as E. coli, as the host and in which the desired protein is expressed by the two-cistron technique. This is a gene expression system comprising two cistrons in sequence and the process yields and accumulates the desired human M-CSF derivative stably in large quantities in host cells.

The production of a human M-CSF derivative of the present invention by said two-cistron method is now described in detail. First, an expression plasmid is prepared which contains two cistrons, namely a gene as the first cistron coding for an appropriate polypeptide and the gene of the invention as the second cistron. It is critical that the plasmid should contain, upstream from the first cistron, a promoter and an SD sequence for the expression of said gene and further contain, downstream from the first cistron but upstream from the second cistron, a synthetic linker which contains an SD sequence for the expression of the second cistron, a termination codon for the first cistron and a initiation codon for the second cistron, as arranged in this order.

The gene to be used as the first cistron may be a synthetic or natural gene insofar as the gene can be expressed in the host. Since the expression of said gene leads to production of a polypeptide different from the human M-CSF derivative of the invention in the same system and this makes it necessary to separate said polypeptide from said derivative, it is desirable that said polypeptide should be hydrophobic and have a molecular weight greatly different from that of the derivative of the invention. It is desired that the above-mentioned first cistron should code for such a polypeptide. As preferred examples of the first cistron, there may be mentioned genes coding for IL-2, IFN-α, -β and -γ, etc. and fragments derived from these genes and coding for about 50 to 100 amino acid residues.

The promoter and SD sequence to be arranged upstream from the first cistron may be per se known ones. Examples of such promoter are the trp promoter, tac promoter, $P_L$ promoter, $P_R$ promoter, lpp promoter, OmpA promoter, lac promoter, etc., among which the trp promoter, $P_L$ promoter, $P_R$ promoter and the like are particularly preferred. Examples of the SD sequence are sequences of 3 to 9 base pairs, such as GGAG and AGGA, that are capable of forming a hydrogen bond with the 3' terminus of 16S rRNA of prokaryotic cells.

The initiation codon and the termination codon to be present in the synthetic linker between the first cistron and the second cistron can be any of the naturally occurring ones. These codons need not be used each in a complete form, for example as TGA and ATG, but can be used in a partly overlapping form, for example as TGATG, TAATG, etc. The expression plasmid for use in the above two-cistron method can be constructed by a usual method.

A method, which can be mentioned as a particularly preferred example, comprises first cleaving an M-CSF gene-containing plasmid with a suitable restriction enzyme, isolating and purifying the resulting M-CSF gene-containing fragment by a usual method separately synthesizing the above-mentioned synthetic linker

7

by a usual method, for example by using a DNA synthesizer, joining the linker to the fragment obtained in the above manner on the upstream side of the M-CSF gene using T4 DNA ligase or the like, and incorporating the resulting DNA fragment into a plasmid containing the first cistron and capable of expressing the same, at a proper position. Alternatively, an expression plasmid suitable for the desired two-cistron system can be prepared from the DNA fragment obtained by the above method and containing the second cistron as joined to the synthetic linker, by joining the first cistron to the upstream end of said fragment, and introducing the resulting DNA fragment into a plasmid having a suitable protein expression system.

The plasmid thus obtained is introduced into suitable host cells for transformation of the cells, whereby the desired transformant can be obtained through the two-cistron method. When such transformant is used, the protein relating to the first cistron and the desired human M-CSF derivative relating to the second cistron can be expressed individually. These products can be analysed or identified by a usual method, for example by SDS-PAGE, Western blotting or the like, and can be separated from each other and purified by the various methods described later herein.

The desired transformant thus obtained can be cultivated by a usual method, whereby the human M-CSF derivative is produced and accumulated. The medium to be used for said cultivation may be one suitably selected from among those usually used for the host cell employed. For cultivating the transformant using E. coli or the like as the host, for example, LB medium, E medium, M9 medium, M63 medium and the like can be used. Various carbon sources, nitrogen sources, inorganic salts, vitamins, natural extracts, physiologically active substances, etc., which are generally known, can be added to these media when required.

In cultivating the above transformant, those cultivation conditions that are suited for the growth of host cells can be employed. In the case of E. coli, for instance, a pH of about 5-8, preferably about 7, and a temperature of about 20-43°C, preferably about 37°C, can be employed.

In the above manner, the desired human M-CSF derivative of the present invention is produced and accumulated in the transformant cells.

This derivative can be isolated and purified by various separation procedures utilizing the physical, chemical or other properties thereof. As typical examples of said procedures, there may be mentioned usual refolding treatment, treatment with a protein precipitating agent, centrifugation, osmotic shock method, ultrasonication, ultrafiltration, molecular sieve chromatography (gel filtration), adsorption chromatography, ion-exchange chromatography, affinity chromatography, high-performance liquid chromatography (HPLC), various other liquid chromatographic techniques, dialysis, and combinations of these. By using such separation procedures, the desired human M-CSF derivative can be produced easily in high yields with high purity on a commercial scale, as shown in the examples.

The thus-obtained human M-CSF derivatives of the present invention are in common in that they have CSF activity although they differ slightly in N-terminal amino acid sequence, molecular weight, etc. depending on the gene used for the production and on the expression system for the expression of said gene and, especially, may have a Met residue corresponding to the initiation codon as added to the N terminus in the process of expression of the derivatives of the invention.

The human M-CSF derivatives obtained in the above manner can be used as various drugs owing to their biological activity. For use as a drug, each human M-CSF derivative of the invention is formulated into pharmacological compositions containing an effective amount of said derivative and a usual pharmacologically acceptable nontoxic carrier. Each composition is given via a route of administration suited for the form thereof. Such compositions are for example, in the form of liquid preparations including solutions, suspensions, emulsions and the like, which are given usually orally, intravenously, subcutaneously, intracutaneously or intramuscularly. Such forms or methods of administration are not limited specifically. The derivatives can also be prepared in other various preparation forms usually used and suited, for example, to oral or nonoral administration. The compositions can be provided also as dry preparations which can be reconstituted to liquids by addition of a suitable carrier. While the dose of each preparation is not limited specifically but can be determined suitably depending on the desired pharmacological effect, the kind of disease, the age and sex of the patient, the severity of disease, etc., the preparation is administered generally at a dose of about 0.001 to about 1 mg/kg/day calculated as the amount of the active component expressed in terms of protein amount. The daily dose may be given either singly or dividedly.

EXAMPLES

In the following, an example of the preparation of a human M-CSF derivative expressed in CHO cells and used for Reference Example 2 is given as Reference Example 1. Then examples of the process for preparing a starting plasmid for the production of the human M-CSF derivatives of the present invention and of the preparation of M-CSFs derived from said plasmid are given as Reference Example 2 and, further, examples of the production of derivatives of the present invention as well as characteristics thereof, etc. are given, for further detailed explanation of the invention.

In the examples, the human M-CSF derivatives are defined as follows. For example, the M-CSF molecule produced by an expression vector coding for the amino acids of position 1 (Val) to position 151 (Thr) of the amino acid sequence defined by formula (1) with E. coli as the host is designated as "E. coli[3-153]M-CSF" and, similarly, the one produced by an expression vector coding for the amino acids of position 1 (Val) to position 212 (Pro) is designated as "E. coli[3-214]M-CSF". The M-CSF derivative obtained in Reference Example 1 as produced by an expression vector coding for a signal peptide comprising a sequence of 32 amino acids and the mature human M-CSF protein comprising a sequence of 522 amino acids with CHO cells as the host is designated as "CHO[-32-522]M-CSF". The numbers in the brackets are due to the fact that the N-terminal region of M-CSF produced in CHO cells has a structure such that two residues (Glu-Glu) additionally occur upstream from the Val of position 1.

The samples obtained in the examples were assayed for CSF activity by the following method.

Method of determining CSF activity

Fetal calf serum (FCS, 20 ml), 30 ml of α-medium and 20 ml of α-medium of 2-fold concentration are mixed together, and the solution is maintained at 37° C. A 23.3-ml portion of the solution is admixed with 10 ml of 1% solution of agar (product of Difco Laboratories) already maintained at 50° C and the mixture is maintained at 37° C.

Separately, bone marrow cells (BMC) collected from the femur of a BALB/c mouse are washed twice with Hanks' solution and then suspended in α-medium to a concentration of $10^7$ cells/ml, and 1 ml of the suspension is added to the agar medium maintained at 37° C. The mixture is thoroughly stirred and then maintained at 37° C. A 0.5-ml portion of the mixture is placed in each well (Tissue Culture Cluster 12, product of Costar Corporation) already containing 50 μl of a test sample, and the resulting mixture is quickly stirred and then allowed to stand at room temperature. On solidification of the agar in each well, the wells are placed in a carbon dioxide gas incubator and further incubated at 37° C for 7 days.

The number of colonies thus produced is counted under a stereoscopic microscope to provide an index for the CSF activity. The CSF activity in units (U/ml) is the value calculated from the above-mentioned colony count according to the following formula (a).

CSF activity in units (U/ml) = (colony count) x (dilution factor) ÷ 1.5     (a)

When observed morphologically and enzymochemically, the colonies that formed in the above were mostly macrophage colonies.

Reference Example 1

Preparation of CHO[-32-522]M-CSF

Using the culture supernatant of the CHO cell clone No. 2, 3-8 [Unexamined Japanese Patent Publication HEI 1-104176] microcarrier-cultured in OPTI-MEM (product of Gibco Laboratories) containing 1% fetal calf serum, the desired homogeneous CHO[-32-522]M-CSF was obtained by the following purification procedure. In the following procedure, the desired protein was detected by the Western blotting method. Said Western blotting was performed using Bio-Rad Labroatories' Transblot cell. The nitrocellulose membrane after transfer was blocked with PBS⁻ containing 1% skim milk, then reacted with a rabbit antiserum against M-CSF and further reacted with peroxidase-labeled goat anti-rabbit antibody (product of Bio-Rad Laboratories). For detecting the M-CSF band, the thus-obtained nitrocellulose membrane was reacted with a solution of the chromogenic substrate 4-chloro-1-naphthol.

(1) ConA-Sepharose chromatography

The above-mentioned CHO cell culture supernatant (69.3 l) was concentrated by ultrafiltration, and a 650-ml portion of the concentrate (842 ml) was used as the starting material and subjected to fractionation with ammonium sulfate. The precipitate fractions obtained by 35%-65% saturation with ammonium sulfate were dissolved in distilled water (1,120 ml) to give a sample solution.

Separately, a column (5 x 25 cm) packed with about 500 ml of ConA-Sepharose gel was equilibrated with 20 mM sodium phosphate buffer (pH 7.4) containing 0.15 M NaCl, and the above sample solution was applied to the column. After thorough washing with the same buffer, elution was carried out with the same buffer containing 0.5 M methyl $\alpha$-D-mannoside. The whole eluate was concentrated by ultrafiltration using a YM-10 membrane, then buffer was exchanged to 20 mM sodium phosphate buffer (pH 7.4), and the resulting solution was subjected, in 7 aliquots, to anion exchange high-performance liquid chromatography under the following conditions.

(2) Anion exchange high-performance liquid chromatography

Column: TSKgel DEAE-5PW (21.5 mm I.D. x 15 cm, product of Tosoh Corporation)
Eluent A: 40 mM Sodium phosphate buffer (pH 7.4)
Eluent B: 40 mM Sodium phosphate buffer (pH 7.4) containing 1.0 M NaCl
Flow rate: 3.0 ml/min.
Fraction volume: 6 ml/tube/2 min.

Concentration gradient:

| Time (min) | B% |
| --- | --- |
| 0 | 0 |
| 10 | 0 |
| 20 | 10 |
| 95 | 30 |
| 100 | 100 |
| 110 | 100 |
| 115 | 0 |
| 130 | 0 |

As a result of the above elution, the desired M-CSF was eluted into fractions Nos. 26-38 (0.18 to 0.25 M NaCl). Said active fractions were pooled, concentrated by ultrafiltration (using a YM-10 membrane) and purified as follows.

(3) Gel filtration high-performance liquid chromatography

The concentrated sample obtained by the above procedure (2) was subjected, in 5 aliquots, to gel filtration high-performance liquid chromatography under the following conditions.
Column: TSKgel G3000SWG (21.5 mm I.D. x 60 cm, product of Tosoh Corporation)
Eluent: 50 mM Sodium phosphate buffer (pH 7.4) containing 0.3 M NaCl
Flow rate: 3.0 ml/min.
Fraction volume: 6 ml/tube/2 min.

As a result of the above gel filtration high-performance liquid chromatography, M-CSF was detected in fractions Nos. 20-27, of which fractions Nos. 24-27 were pooled, concentrated by ultrafiltration and subjected to the following purification procedure.

(4) TSKgel Ether-5PW high-performance liquid chromatography

The concentrated sample (12 ml) obtained by the above procedure (3) was purified, in 8 aliquots, under the following conditions.

Prior to injection into the column, the above-mentioned concentrate was admixed with 80% ammonium sulfate-saturated 20 mM sodium phosphate buffer (pH 7.4) and then subjected to chromatography.
Column: TSKgel Ether-5PW (7.5 mm I.D. x 75 mm, product of Tosoh Corporation)
Eluent A: 20 mM Sodium phosphate buffer (pH 7.4)
Eluent B: 40% Ammonium sulfate-saturated 20 mM sodium phosphate (pH 7.4)
Flow rate: 1.0 ml/min.
Fraction volume: 2 ml/tube/2 min.

Concentration gradint:

| Time (min) | B% |
| --- | --- |
| 0 | 100 |
| 5 | 100 |
| 65 | 0 |
| 80 | 0 |
| 85 | 100 |
| 100 | 100 |

As a result of the above chromatography, M-CSF was detected in fractions Nos. 15-18 (22%-17% saturated ammonium sulfate concentrations). Fractions having the same fraction number were respectively pooled and each pool was subjected, in 3 or 4 aliquots, to reversed-phase high-performance liquid chromatography.

(5) TSKgel Phenyl-5PWRP reversed-phase high-performance liquid chromatography

Column:              TSKgel Phenyl-5PWPP (7.5 mm I.D. x 75 mm, product of Tosoh Corporation)
Eluent A:            0.1% TFA
Eluent B:            n-Propanol: 1% TFA (9:1)
Flow rate:           0.8 ml/min.
Fraction volume:     1.6 ml/tube/2 min.

Concentration gradient:

| Time (min) | B% |
| --- | --- |
| 0 | 0 |
| 5 | 0 |
| 10 | 25 |
| 50 | 45 |
| 60 | 100 |
| 70 | 100 |
| 75 | 0 |
| 95 | 0 |

As a result of the above chromatography, M-CSF was detected in two consecutive fractions out of fractions Nos. 5-8 (32%-34% n-propanol), although the fraction numbers of the two varied a little depending on the sample and experiment.

The fractionation was carried out using tubes already containing 200 µl of 0.8 M disodium phosphate per tube. The M-CSF fractions were concentrated to dryness by centrifugation tube by tube on a concentrator (product of Tomy Seiko Co., Ltd.) and dissolved in 500 µl per tube of distilled water and the resulting solutions were subjected to experimentation.

(6) SDS-PAGE of CHO[-32-522]M-CSF

According to the method of Laemmli [Laemmli, U. K., Nature, 277, 680 (1970)], the CHO[-32-522]M-CSF obtained by the above procedure (5) was admixed with Laemmli's sample buffer [either of the one containing 2-mercaptoethanol (2-ME$^+$) and the one free of it (2-ME$^-$)], the respective mixtures were heat-treated at 95°C for 10 minutes and then subjected to SDS-PAGE using mini slab gels (gel concentration 15%). Prestained markers (product of Bio-Rad Laboratories) were used as molecular weight markers. For staining, Silver Stain (product of Wako Pure Chemical Industries) was used.

As a result, under non-reducing conditions (state of 2-ME$^-$), a smeared band was detected over the molecular weight range of 62,000-115,000 with a main component at a molecular weight of about 85,000 and, under reducing conditions (state of 2-ME$^+$), a smear band was detected over 39,000-46,000 with a main component at 43,000.

(7) N-Terminal region amino acid sequence of CHO[-32-522]M-CSF

11

The N-terminal region amino acid sequence of the CHO[-32-522]M-CSF obtained by the above procedure (5) was determined using a gaseous phase sequencer (product of Applied Biosystems).

As a result, the sequence of the N-terminal 10 amino acids was confirmed to be as follows. The amino acid (X') in cycle 7 could not be identified but was estimated as Cys based on the gene structure.

Glu-Glu-Val-Ser-Glu-Tyr-X'-Ser-His-Met-

Reference Example 2

[1] Preparation of plasmid ptrpIL-2X-M-CSF101

The plasmid pcDM-CSF11-185 [prepared from the plasmid pcDM-CSF11 containing the M-CSF gene (λcM11 cDNA, about 2.5 kb) (cf. Unexamined Japanese Patent Publication HEI 1-104176)] was digested with the restriction enzymes ScaI and BamHI, and a ScaI-BamHI DNA fragment (about 450 bp) was isolated and purified by agarose gel electrophoresis. Then, the synthetic linker (A) shown below was ligated to the ScaI cleavage site of the above-obtained DNA fragment using T4 DNA ligase to give an XbaI-BamHI DNA fragment (about 480 bp) having an XbaI (restriction enzyme) cleavage site on the ScaI cleavage site side.

Synthetic linker (A):

```
                2nd SD                    ter
  5'- CTA GAA CGG AGG ACT CAT TG|ATG| GTA TCA GAA  T-3'
          TT·GCC TCC TGA GTA ACTAC CAT AGT CTT  A
      XbaI                        start            ScaI
                                 (Box)
```

The thus-obtained DNA fragment was inserted into the human IL-2 expression plasmid ptrpIL-2D8Δ - (Unexamined Japanese Patent Publication SHO 63-12958) between the XbaI and BamHI cleavage sites, whereby the desired plasmid ptrpIL-2X-M-CSF101 was obtained.

A transformant obtained by transforming Escherichia coli HB101 with said plasmid has been deposited, under the name of Escherichia coli HB101/ptrp IL-2X-M-CSF101, in the Fermentation Research Institute, Agency of Industrial Science and Industry with the deposition number of FERM BP-2226 (E. coli [3-153] FERM BP-2226] since December 26, 1988.

[2] Isolation and purification of E.coli[3-153] M-CSF

(1) Preparation of M-CSF fraction from Escherichia coli

To 1.5 g (wet weight) of an E. coli strain SG21058 harboring the plasmid ptrpIL-2X-M-CSF101 obtained by the above procedure [1] was added 50 ml of 50 mM Tris-hydrochloride buffer (pH 7.0) containing 0.5 M sucrose, and the mixture was thoroughly stirred. Then, 6 ml of 2 mg/ml lysozyme was added, 4 ml of 0.14 M EDTA was further added and, after 15 minutes of stirring at 4° C, the mixture was centrifuged at 10,000 revolutions/minute for 20 minutes.

The supernatant was discarded, and the pellet was washed with the same buffer (50 mM Tris-hydrochloride containing 0.5 M sucrose, pH 7.0) and subjected to centrifugation, which was carried out similarly at 10,000 revolutions/minute for 20 minutes and gave spheroplasts as a pellet. The pellet was suspended in 50 ml of 50 mM Tris-hydrochloride (pH 7.0) and the suspension was subjected to sonication at 20 KHz for 10 minutes and then centrifuged at 10,000 revolutions/minute for 20 minutes. The pellet was washed with the same buffer (50 mM Tris-hydrochloride, pH 7.0) and again centrifuged under the same conditions to give an M-CSF fraction as a pellet.

(2) Refolding of M-CSF from M-CSF fraction

To the M-CSF fraction obtained by the above procedure (1) was added 100 ml of 50 mM Tris-hydrochloride (pH 7.0) containing 7.0 M guanidine hydrochloride, and the mixture was solubilized by agitating with a stirrer at 4°C for 1 hour. The solution was slowly added dropwise into a beaker already containing 300 ml of 10 mM Tris-hydrochloride (pH 8.5) (agitated with stirrer) and, after completion of the dropping, the mixture was thoroughly dialyzed against 10 mM Tris-hydrochloride (pH 8.5) at 4°C and then centrifuged at 10,000 revolutions/minute for 20 minutes. The precipitate was discarded and the supernatant was recovered.

The refolded M-CSF is present in the thus-obtained supernatant.

(3) Purification of M-CSF

The M-CSF obtained in the above manner was purified as follows.

(3-1) Gel filtration high-performance liquid chromatography

The supernatant obtained by the above procedure (2) was concentrated by ultrafiltration device (product of Amicon; membrane: YM-10 membrane, product of Amicon), and the concentrate was passed through a 0.45 μm Millipore filter and then subjected to gel filtration high-performance liquid chromatography under the following conditions.

| | |
|---|---|
| Column: | TSKgel G 3000SW (60 cm x 21.5 mm I.D., product of Tosoh Corporation) |
| Eluent: | 50 mM sodium phosphate buffer (pH 6.8) containing 0.3 M NaCl |
| Flow rate: | 3.0 ml/min. |
| Fraction volume: | 6 ml/tube/2 min. |

In the above procedure, judging from the elution positions of standard proteins for gel filtration HPLC (product of Oriental Yeast Co., Ltd.), namely glutamate dehydrogenase (molecular weight 290,000), lactate dehydrogenase (molecular weight 142,000), enolase (molecular weight 67,000) and adenylate kinase (molecular weight 32,000), the molecular weight of M-CSF was estimated to be 32,000.

The active fractions were collected and buffer exchange was performed against 40 mM sodium borate (pH 8.0) by ultrafiltration as mentioned above.

(3-2) TsKgel DEAE-5PW ion exchange high-performance liquid chromatography

The active eluate fraction obtained by the above procedure (3-1) was subjected to TSKgel ion exchange high-performance liquid chromatography under the following conditions.

| | |
|---|---|
| Column: | TSKgel DEAE-5PW (7.5 mm I.D. x 75 mm, product of Tosoh Corporation) |
| Eluent A: | 40 mM Sodium borate buffer (pH 8.0) containing 5% methanol |
| Eluent B: | 40 mM Sodium borate buffer (pH 8.0) containing 1.0 M NaCl and 5% methanol |
| Flow rate: | 1.0 ml/min. |
| Fraction volume: | 1.0 ml/tube/min. |

| Concentration gradient: | Time (min) | B% |
|---|---|---|
| | 0 | 0 |
| | 7 | 0 |
| | 42 | 30 |
| | 47 | 100 |
| | 52 | 100 |
| | 57 | 0 |
| | 62 | 0 |

Judging from the results (elution pattern) of the above TSKgel DEAE-5PW ion exchange high-performance liquid chromatography, the peaks observed with fractions Nos. 42-43 (NaCl concentration 0.23-0.25 M) corresponded to M-CSF and said peaks were collected. Thus, purified M-CSF was obtained from Escherichia coli.

13

Example 1

(1) Preparation of ptrpIL-2X-M-CSF201

The plasmid ptrpIL-2X-M-CSF101 was cleaved with the restriction enzymes BstEII and SalI and a 4.7 kb DNA fragment (I) containing the IL-2 and M-CSF DNA portions was obtained.

Separately, the plasmid pcDM-CSF11 was cleaved with the restriction enzyme EcoRI. The cleavage ends were blunt-ended using DNA polymerase I Klenow fragment and then a SalI linker [5'-pGGTCGACC$_{OH}$-3') (product of New England Biolabs) was ligated thereto. This ligation product was cleaved with the restriction enzymes SalI and BstEII to give a BstEII-SalI fragment (II) of about 1.1 kb.

The above-mentioned DNA fragments (I) and (II) were ligated together using T4 DNA ligase and the ligation product was used to transform competent cells of E. coli HB101 to give a desired transformant E. coli HB101 strain carrying the plasmid ptrpIL-2X-M-CSF201.

The thus-obtained plasmid ptrpIL-2X-M-CSF201 encodes two polypeptides within the transcription unit under the control of the E. coli tryptophan promoter. One is a 65-amino-acid polypeptide composed of methionine (translation initiation), amino terminal 60 amino acids of human IL-2 and 4 amino acids encoded by the synthetic DNA linker, and the other is a polypeptide comprising a human M-CSF derivative composed of Met (translation initiation) and those 520 amino acids covering the position-1 amino acid (Val) to the position-520 amino acid (Val) of the amino acid sequence defined by formula (1).

In such two-cistron expression system, the translation of the second cistron is initiated by binding of a ribosome to the second SD sequence located in the synthetic DNA linker.

(2) Preparation of ptrpIL-2X-M-CSF202

The plasmid ptrpIL-2X-M-CSF201 was cleaved with the restriction enzymes NcoI and SalI and an NcoI-SalI DNA fragment of about 4.8 kb was recovered. Both ends of this DNA fragment was ligated to each other using synthetic oligonucleotides [5'-CATGGCCTGATAAG-3' and 5'-TCGACTTATCAGGC-3'] with T4 DNA ligase. Competent cells of E. coli HB101 were transformed with the ligation product and a desired transformant carrying the plasmid ptrpIL-2X-M-CSF202 was obtained.

The ptrpIL-2X-M-CSF202 obtained encodes a polypeptide comprising a human M-CSF derivative composed of Met (translation initiation) and those 182 amino acids covering the position-1 amino acid (Val) to the position-182 amino acid (Ala) of the amino acid sequence defined by formula (1) in the second cistron within the transcription unit under the control of the E. coli tryptophan promoter.

(3) Preparation of ptrpIL-2X-M-CSF203

The plasmid ptrpIL-2X-M-CSF201 was cleaved with the restriction enzymes BamHI and SalI and a BamHI-SalI DNA fragment of about 4.9 kb was recovered. Both ends of this DNA fragment were ligated to each other using synthetic oligonucleotides [5'-GATCCATGATAAG-3' and 5'-TCGACTTATCATG-3'] with T4 DNA ligase and the product was used to transform competent cells of E. coli HB101, whereby a desired transformant carrying the plasmid ptrpIL-2X-M-CSF203 was obtained.

The thus-obtained plasmid ptrpIL-2X-M-CSF203 encodes a polypeptide comprising a human M-CSF derivative composed of Met (translation initiation) and those 212 amino acids covering the position-1 amino acid (Val) to the position-212 amino acid (Pro) of the amino acid sequence defined by formula (1) in the second cistron within the transcription unit under the control of the E. coli tryptophan promoter.

The above-mentioned E. coli HB101 strain carrying the plasmid ptrpIL-2X-M-CSF203 has been deposited, under the designation "Escherichia coli HB101/ptrpIL-2X-M-CSF203", with the Fermentation Research Institute, Agency of Industrial Science and Technology under the deposition number FERM P-11053 as of October 18, 1989.

(4) Preparation of ptrpIL-2X-M-CSF204

The plasmid ptrpIL-2X-M-CSF201 was cleaved with the restriction enzymes SmaI and SalI and a SmaI-SalI DNA fragment of about 5.0 kb was recovered. Both ends of this DNA fragment were ligated to each other using synthetic oligonucleotides [5'-GGGTGATAAG-3' and 5'-TCGACTTATCACCC-3'] with T4 DNA ligase and the product was used to transform competent cells of E. coli HB101 to give a desired transformant carrying the plasmid ptrpIL-2X-M-CSF204.

The plasmid ptrpIL-2X-M-CSF204 thus obtained encodes a polypeptide comprising a human M-CSF derivative composed of Met (translation initiation) and those 256 amino acids covering the position-1 amino acid (Val) to the position-256 amino acid (Gly) of the amino acid sequence defined by formula (1) in the second cistron within the transcription unit under the control of the E. coli tryptophan promoter.

(5) Preparation of ptrpIL-2X-M-CSF205

The plasmid ptrpIL-2X-M-CSF201 was cleaved with the restriction enzymes SphI and SalI and an SphI-SalI DNA fragment of about 5.1 kb was recovered. Both ends of this DNA fragment were ligated to each other using synthetic oligonucleotides [5'-CAGTGATAAG-3' and 5'-TCGACTTATCACTGCATG-3'] with T4 DNA ligase and the product was used to transform competent cells of E. coli HB101 to give a desired transformant carrying the plasmid ptrp-IL-2X-M-CSF205.

The thus-obtained plasmid ptrpIL-2X-M-CSF205 encodes a polypeptide comprising a human M-CSF derivative composed of Met (translation initiation) and those 300 amino acids covering the position-1 amino acid (Val) to the position-300 amino acid (Gln) of the amino acid sequence defined by formula (1) in the second cistron within the transcription unit under the control of the E. coli tryptophan promoter.

(6) Preparation of ptrpIL-2X-M-CSF206

The plasmid ptrpIL-2X-M-CSF201 was cleaved with the restriction enzymes KpnI and SalI and a KpnI-SalI DNA fragment of about 5.2 kb was recovered. Both ends of this DNA fragment were ligated to each other using synthetic oligonucleotides [5'-CGCCTGATAAG-3' and 5'-TCGACTTATCAGGCGGTAC-3'] with T4 DNA ligase and the product was used to transform competent cells of E. coli HB101 to give a desired transformant carrying the plasmid ptrpIL-2X-M-CSF206.

The thus-obtained plasmid ptrpIL-2X-M-CSF206 encodes a polypeptide comprising a human M-CSF derivative composed of Met (translation initiation) and those 332 amino acids covering the position-1 amino acid (Val) to the position-332 amino acid (Ala) of the amino acid sequence defined by formula (1) in the second cistron within the transcription unit under the control of the E. coli tryptophan promoter.

(7) Expression of M-CSF derivatives

The plasmids respectively obtained by the above procedures (1) to (6) were each introduced into the E. coli strain SG21058 [J. Bacteriol., 164, 1124-1135 (1985)] by the transformation method to give transformants (respectively designated as E. coli SG21058/ptrpIL-2X-M-CSF201, E. coli SG21058/ptrpIL-2X-M-CSF202, E. coli SG21058/ptrpIL-2X-M-CSF203, E. coli SG21058/ptrpIL-2X-M-CSF204, E. coli SG21058/ptrpIL-2X-M-CSF205 and E. coli SG21058/ptrp-IL-2X-M-CSF206].

These transformants were cultivated in LB medium (T. Maniatis, E. F. Fritsch and J. Sambrook, Molecular Cloning, A Laboratory Manual, p. 440 (1982), Cold Spring Harbor Laboratory) containing 50 μg/ml ampicillin overnight at 37°C. A 0.5-ml portion of each culture was added to 50 ml of M9 medium (see the reference cited above) supplemented with 1% casamino acid, 5 μg/ml thiamine hydrochloride, 20 μg/ml L-cysteine and 50 μg/ml ampicillin with the glucose concentration adjusted to 0.4%, shake culture was performed at 37°C for 8 hours, then cells were recovered by centrifugation (5,000 rpm), and the protein produced was analyzed by SDS-PAGE and Western blotting.

The Western blotting was performed using Bio-Rad's Transblot cells. Nitrocellulose membranes after transfer were blocked with PBS⁻ containing 1% bovine serum albumin, then reacted with a rabbit antiserum against M-CSF and further reacted with peroxidase-labeled goat anti-rabbit antibody (product of Bio-Rad Laboratories). M-CSF band detection was performed by reacting the thus-obtained nitrocellulose membranes with a solution of the chromogenic substrate 4-chloro-1-naphthol.

In E. coli SG21058/ptrpIL-2X-M-CSF202, E. coli SG21058/ptrpIL-2X-M-CSF203, E. coli SG21058/ptrpIL-2X-M-CSF204, E. coli SG21058/ptrpIL-2X-M-CSF205 and E. coli SG21058/ptrpIL-2X-M-CSF206, the M-CSFs encoded in the second cistrons were respectively detected at positions corresponding to the expected molecular weights.

(8) Isolation and purification of M-CSF derivative (E.coli[3-214]-M-CSF)

To 10 g (wet weight) of E. Coli SG21058 cells harboring the plasmid ptrpIL-2X-CSF203 obtained by the above procedure (3) was added 50 mM Tris-hydrochloride buffer (pH 7.0) to make the whole volume 100 ml, followed by thorough stirring. Then, 4 ml of 6 mg/ml lysozyme and 8 ml of 0.14 M EDTA were added, and the mixture was stirred at 4°C for 15 minutes, then sonicated (200 KHz, 2 minutes, 200 W) and further centrifuged at 10,000 revolutions/minute for 20 minutes. The pellet obtained was thoroughly washed with 50 mM Tris-hydrochloride (pH 7.0) containing 2% Triton X-100 and again subjected to centrifugation under the same conditions to give an M-CSF fraction as the pellet.

To the thus-obtained M-CSF fraction was added 20 ml of 50 mM Tris-hydrochloride (pH 7.0) containing 7.0 M guanidine hydrochloride and 50 mM mercaptoethanol, and the mixture was stirred at room temperature for 4 hours for reducing, denaturing and solubilizing the protein. This solution was gradually added dropwise into a breaker already containing 2,000 ml of 50 mM Tris-hydrochloride (pH 8.5) containing 0.5 mM reduced-form glutathione, 0.1 mM oxidized-form glutathione and 2 M urea (agitated with stirrer) for 100-fold dilution. After completion of the dropping, the dilution was allowed to stand at 4°C for at least 2 days and then centrifuged at 3,000 revolutions/minute for 30 minutes. The pellet was removed and the supernatant (2 liters) was recovered.

The refolded M-CSF was present in the thus-obtained supernatant.

One liter of the centrifugation supernatant obtained by the above procedure was concentrated using an ultrafiltration device (product of Amicon, with a YM-10 membrane (Amicon)), and the concentrate was centrifuged at 10,000 revolutions/minute for 20 minutes. The pellet was removed and the supernatant was recovered. To this supernatant was added ammonium sulfate in an amount sufficient for 30% saturation. The resultant mixture was again centrifuged in the same manner and the supernatant was subjected to hydrophobic interaction chromatography under the following conditions.

| | |
|---|---|
| Column: | TSKgel Phenyl 5PW (7.5 mm I.D. x 75 mm, product of Tosoh Corporation) |
| Eluent A: | 30% Ammonium sulfate-saturated 50 mM sodium phosphate buffer (pH 7.4) |
| Eluent B: | 50 mM Sodium phosphate buffer (pH 7.4) |
| Flow rate: | 1.0 ml/min. |
| Fraction volume: | 1 ml/tube/min. |

Concentration gradient:

| Time (min.) | B% |
|---|---|
| 0 | 0 |
| 7 | 0 |
| 40 | 100 |
| 45 | 100 |
| 50 | 0 |
| 60 | 0 |

As a result of the above procedure, an M-CSF activity was eluted in fractions Nos. 40-42 (ammonium sulfate concentration 8-6% saturation). The active fractions were collected, subjected to buffer exchange for 50 mM sodium phosphate (pH 7.4) by ultrafiltration mentioned above, and then subjected to DEAE-5PW ion exchange high-performance liquid chromatography under the following conditions.

| | |
|---|---|
| Column: | DEAE-5PW (7.5 mm I.D. x 75 mm, product of Tosoh Corporation) |
| Eluent A: | 50 mM Sodium phosphate buffer (pH 7.4) |
| Eluent B: | 50 mM Sodium phosphate buffer (pH 7.4) containing 1.0 M NaCl |
| Flow rate: | 1.0 ml/min. |
| Fraction volume: | 1.0 ml/tube/min. |

Concentration gradient:

| Time (min) | B% |
|---|---|
| 0 | 0 |
| 7 | 0 |
| 42 | 30 |
| 47 | 100 |
| 52 | 100 |
| 57 | 0 |
| 62 | 0 |

From the result (elution pattern) of the above DEAE-5PW ion exchange high-performance liquid chromatography, the peaks observed in fractions Nos. 31 and 32 (NaCl concentration 0.18-0.20 M) corresponded to M-CSF. Said peaks were collected and a human M-CSF derivative of the present invention was obtained.

In the above purification steps, the activity of the human M-CSF derivative of the invention, the protein weight, the specific activity and the degree of purification were determined. The results are shown below in Table 1.

Table 1

| Purification step | Volume (ml) | Activity (unit) | Protein weight (mg) | Specific activity (unit/mg protein) | Degree of purification (times) |
|---|---|---|---|---|---|
| Refolding liquid | 1000 | $4.62 \times 10^7$ | 110 | $4.20 \times 10^5$ | 1.0 |
| 30% Ammonium Sulfate supernatant | 45 | $3.93 \times 10^7$ | 10.1 | $3.89 \times 10^6$ | 9.3 |
| Phenyl 5-PW HPLC | 3.6 | $1.50 \times 10^7$ | 1.82 | $8.24 \times 10^6$ | 19.6 |
| DEAE-5PW HPLC | 2.0 | $7.84 \times 10^6$ | 0.514 | $1.53 \times 10^7$ | 36.4 |

(9) SDS-PAGE of M-CSF derivative (E.coli[3-214]-M-CSF)

According to the method of Laemmli [Laemmli, U. K., Nature, 277, 680 (1970)], the sample purified in the above manner was dissolved in each of Laemmli's sample buffer [2-ME$^+$ and 2-ME$^-$] and each solution was heat-treated at 95°C for 5 minutes and then subjected to electrophoresis using a microslab gel [apparatus: product of Marisoru Sangyo K.K. or Daiichi Chemical Co., Ltd.]. Prestained markers (product of Bio-Rad Laboratories) were used as molecular weight markers and Coomassie Brilliant Blue R-250 was used for staining.

As a result, the molecular weight of E.coli[3-214]-M-CSF was found to be about 52,500 in the 2-ME⁻ state and about 26,900 in the 2-ME⁺ state and the electrophoresis gave a single band at each of said positions.

Example 2

(1) Preparation of plasmid ptrpIL-2X-M-CSF109

The plasmid pcDM-CSF11-185 [prepared from the plasmid pcDM-CSF 11 containing the M-CSF gene (λcM11 cDNA, about 2.5 kb) (cf. Unexamined Japanese Patent Publication HEI 1-104176)] was digested with the restriction enzymes ScaI an BamHI and a ScaI-BamHI DNA fragment (about 450 bp) was isolated and purified by agarose gel electrophoresis.

Then, a synthetic linker (B) shown below was ligated to the ScaI cleavage site of the DNA fragment obtained using T4 DNA ligase, whereby an XbaI-BamHI DNA fragment (about 480 bp) having an XbaI (restriction enzyme) cleavage site on the ScaI cleavage site side was obtained.

Synthetic linker (B):

$$\underset{\text{2nd SD}}{\underline{\phantom{xxxxxx}}} \qquad \underset{\text{ter}}{\underline{\phantom{xx}}}$$

5'-CTA GAA CGG AGG ACT CAT TG|ATG| TCA GAA T-3'

TT GCC TCC TGA GTA ACTAC AGT CTT A

start

(Box)

The thus-obtained DNA fragment was inserted into the human IL-2 expression plasmid ptrpIL-2D8Δ (cf. Unexamined Japanese Patent Publication SHO 63-12958) between the XbaI and BamHI cleavage sites to give a desired plasmid, ptrpIL-2X-M-CSF109.

The plasmid ptrpIL-2X-M-CSF109 thus obtained encodes two polypeptides within the transcription unit under the control of the E. coli tryptophan promoter. One is a 65-amino-acid polypeptide composed of Met (translation initiation), amino terminal 60 amino acids of human IL-2 and 4 amino acids resulting from the synthetic linker DNA sequence, and the other is a polypeptide composed of Met (translation initiation) and 150 amino acids comprising the position-2 amino acid (Ser) to the position-151 amino acid (Thr) of the amino acid sequence defined by formula (1).

(2) Preparation of ptrpIL-2X-M-CSF402

The plasmid ptrpIL-2X-M-CSF109 was cleaved with the restriction enzymes BstEII and ScaI and a DNA fragment of about 1.5 kb was recovered.

Separately, the plasmid ptrpIL-2X-M-CSF202 was cleaved in the same manner with the restriction enzymes BstEII and ScaI and a DNA fragment of about 3.3 kb was recovered.

Both the above DNA fragments were ligated to each other using T4 DNA ligase and the ligation mixture was used to transform competent cells of E. coli HB101 to give an E. coli HB101 transformant carrying the desired plasmid ptrpIL-2X-M-CSF402.

The thus-obtained plasmid ptrpIL-2X-M-CSF402 encodes, in the second cistron within the transcription unit under the control of the E. coli tryptophan promoter, a polypeptide comprising a human M-CSF derivative composed of Met (translation initiation) and 181 amino acids from the position-2 amino acid (Ser) to the position-182 amino acid (Ala) of the amino acid sequence defined by formula (1).

### (3) Preparation of ptrpIL-2X-M-CSF403

The plasmid ptrpIL-2X-M-CSF109 was cleaved with the restriction enzymes BstEII and ScaI and a DNA fragment of about 1.5 kb was recovered.

Separately, the plasmid ptrpIL-2X-M-CSF203 was cleaved in the same manner with the restriction enzymes BstEII and ScaI and a DNA fragment of about 3.4 kb was recovered.

Both the above DNA fragments were ligated to each other using T4 DNA ligase and the ligation mixture was used to transform competent cells of E. coli HB101, whereby an E. coli HB101 transformant carrying the desired plasmid ptrpIL-2X-M-CSF403 was obtained.

The thus-obtained plasmid ptrpIL-2X-M-CSF403 encodes, in the second cistron within the transcription unit under the control of the E. coli tryptophan promoter, a polypeptide comprising a human M-CSF derivative composed of Met (translation initiation) and 211 amino acids from the position-2 amino acid (Ser) to position-212 amino acid (Pro) of the amino acid sequence defined by formula (1).

### (4) Preparation of ptrpIL-2X-M-CSF404

The plasmid ptrpIL-2X-M-CSF109 was cleaved with the restriction enzymes BstEII and ScaI and a DNA fragment of about 1.5 kb was recovered.

Separately, the plasmid ptrpIL-2X-M-CSF204 was cleaved in the same manner with the restriction enzymes BstEII and ScaI and a DNA fragment of about 3.5 kb was recovered.

Both the above DNA fragments were ligated to each other using T4 DNA ligase and the ligation mixture was used to transform competent cells of E. coli HB101, whereby an E coli HB101 transformant carrying the desired plasmid ptrpIL-2X-M-CSF404 was obtained.

The thus-obtained plasmid ptrpIL-2X-M-CSF404 encodes, in the second cistron within the transcription unit under the control of the E. coli tryptophan promoter, a polypeptide comprising a human M-CSF derivative composed of Met (translation initiation) and 255 amino acids from the position-2 amino acid (Ser) to the position-256 amino acid (Gly) of the amino acid sequence defined by formula (1).

### (5) Preparation of ptrpIL-2X-M-CSF405

The plasmid ptrpIL-2X-M-CSF109 was cleaved with the restriction enzymes BstEII and ScaI and a DNA fragment of about 1.5 kb was recovered.

Separately, the plasmid ptrpIL-2X-M-CSF205 was cleaved in the same manner with the restriction enzymes BstEII and ScaI and a DNA fragment of about 3.6 kb was recovered.

Both the above DNA fragments were ligated to each other using T4 DNA ligase and the ligation mixture was used to transform competent cells of E. coli HB101, whereby an E. coli HB101 transformant carrying the desired plasmid ptrpIL-2X-M-CSF405 was obtained.

The thus-obtained plasmid ptrpIL-2X-M-CSF405 encodes, in the second cistron within the transcription unit under the control of the E. coli tryptophan promoter, a polypeptide comprising a human M-CSF derivative composed of Met (translation initiation) and 299 amino acids from the position-2 amino acid (Ser) to the position-300 amino acid (Gln) of the amino acid sequence defined by formula (1).

### (6) Preparation of ptrpIL-2X-M-CSF406

The plasmid ptrpIL-2X-M-CSF109 was cleaved with the restriction enzymes BstEII and ScaI and a DNA fragment of about 1.5 kb was recovered.

Separately, the plasmid ptrpIL-2X-M-CSF206 was cleaved in the same manner with the restriction enzymes BstEII and ScaI and a DNA fragment of about 3.7 kb was recovered.

Both the above DNA fragments were ligated to each other using T4 DNA ligase and the ligation mixture was used to transform competent cells of E. coli HB101, whereby an E coli HB101 transformant carrying the desired plasmid ptrpIL-2X-M-CSF406 was obtained.

The thus-obtained plasmid ptrpIL-2X-M-CSF406 encodes, in the second cistron within the transcription unit under the control of the E. coli tryptophan promoter, a polypeptide comprising a human M-CSF derivative composed of Met (translation initiation) and 331 amino acids from the position-2 amino acid (Ser) to the position-332 amino acid (Ala) of the amino acid sequence defined by formula (1).

(7) Expression of M-CSF derivatives

The plasmids respectively obtained by the above procedures (1)-(6) were each introduced into the E. coli strain SG21058 [J. Bacteriol., 164 1124-1135 (1985)] by the transformation method to give transformants [respectively designated as E. coli SG21058/ptrpIL-2X-M-CSF109, E. coli SG21058/ptrpIL-2X-M-CSF402, E. coli SG21058/ptrpIL-2X-M-CSF403, E. coli SG21058/ptrpIL-2X-M-CSF404, E. coli SG21058/ptrpIL-2X-M-CSF405 and E. coli SG21058/ptrpIL-2X-M-CSF406].

These transformants were cultured in LB medium (T. Maniatis, E. F. Fritsch and J. Sambrook, Molecular Cloning, A Laboratory Manual, p. 440 (1982), Cold Spring Harbor Laboratory) containing 50 μg/ml ampicillin overnight at 37°C. A 0.5-ml portion of each culture was added to 50 ml of M9 medium (cf. the reference cited above) with 1% casamino acids, 5 μg/ml thiamine hydrochloride, 20 μg/ml L-cysteine and 50 μg/ml ampicillin added and the glucose concentration adjusted to 0.4% and, after 8 hours of shake culture at 37°C, cells were recovered by centrifugation (5,000 rpm), and the protein produced was analyzed, in the same manner as in Example 1-(7), by SDS-PAGE and Western blotting.

As a result, the M-CSFs each encoded in the second cistron were detected in positions corresponding to the respective expected molecular weights for E. coli SG21058/ptrpIL-2X-M-CSF109, E. coli SG21058/ptrpIL-2X-M-CSF402, E. coli SG21058/ptrpIL-2X-M-CSF403, E. coli SG21058/ptrpIL-2X-M-CSF404, E. coli SG21058/ptrpIL-2X-M-CSF405 and E. coli SG21058/ptrpIL-2X-M-CSF406.

(8) Isolation and purification of M-CSF derivative (E.coli[4-153]-M-CSF)

(a) Preparation of M-CSF fraction from E. coli

To 7.5 g (wet weight) of the E. coli SG21058 strain carrying the plasmid ptrpIL-2X-M-CSF109 obtained by the above procedure (1) was added 50 mM Tris-hydrochloride buffer (pH 7.0) to make the whole volume 100 ml, followed by thorough stirring. Then, 6 ml of 2 mg/ml lysozyme and then 4 ml of 0.14 M EDTA were added, and the mixture was stirred at 4°C for 15 minutes, then sonicated (20 KHz, 10 minutes, 200 W) and further centrifuged at 10,000 revolutions/minute for 20 minutes to give a pellet. This was further washed with a buffer for washing (50 mM Tris-hydrochloride buffer containing 2% Triton X-100, pH 7.0) and again centrifuged under the same conditions. After two repetitions of this procedure, an M-CSF fraction (pellet) was obtained.

(b) Refolding of M-CSF from M-CSF fraction

To the M-CSF fraction obtained by the above procedure (a) was added 20 ml of 50 mM Tris-hydrochloride buffer (pH 7.0) containing 7 M guanidine hydrochloride and 25 mM 2-mercaptoethanol, and the mixture was stirred at room temperature for at least 4 hours for reducing, denaturing and solubilizing the protein. This solution was gradually added dropwise into a beaker already containing 2,000 ml of 50 mM Tris-hydrochloride buffer (pH 8.5) containing 0.5 mM reduced-form glutathione, 0.1 mM oxidized-form glutatione and 2 M urea (agitated with stirrer) and the resultant mixture was allowed to stand at 4°C for at least 2 days. The solution was then centrifuged at 3,500 revolutions/minute for 30 minutes, and the pellet was removed and the supernatant was recovered.

The refolded M-CSF was present in the thus-obtained supernatant.

(c) Purification of M-CSF

The centrifugation supernatant obtained by the above procedure (b) was purified in the following manner.

(c-1) Hydrophobic high-performance liquid chromatography

The above centrifugation supernatant was concentrated by ultrafiltration device (product of Amicon, with a YM-10 membrane (Amicon) used), ammonium sulfate was added to the concentrate to make a 30%-saturated solution, and the resultant solution was centrifuged at 10,000 revolutions/minute for 20 minutes. The pellet was removed and the supernatant was recovered. This supernatant was passed through a 0.45-μm Millipore filter and then subjected, in two portions, to hydrophobic interaction chromatography under the following conditions.

Column:          TSKgel Phenyl 5PW (7.5 mm I.D. x 75 mm, product of Tosoh Corporation)

Eluent A:        30% Ammonium sulfate-saturated 40 mM sodium phosphate buffer (pH 7.4)
Eluent B:        40 mM Sodium phosphate buffer (pH 7.4)
Flow rate:      1.0 ml/min.
Fraction volume:  1 ml/tube/min.

| Concentration gradient: | Time (min) | B% |
|---|---|---|
| | 0 | 0 |
| | 7 | 0 |
| | 40 | 100 |
| | 45 | 100 |
| | 50 | 0 |

As a result, an M-CSF activity was eluted in fractions corresponding to the ammonium sulfate concentrations of 11-6%. Said active fractions were collected and subjected to buffer exchange for 40 mM sodium phosphate (pH 7.4) by ultrafiltration mentioned above.

(c-2) Anion exchange high-performance liquid chromatography

The fraction obtained by the above procedure (c-1) was subjected, in 4 portions, to DEAE-5PW anion exchange high-performance liquid chromatography under the following conditions.

Column:         TSKgel DEAE-5PW (7.5 mm I.D. x 75 mm, product of Tosoh Corporation)
Eluent A:        50 mM Sodium phosphate buffer (pH 7.4)
Eluent B:        50 mM Sodium phosphate buffer (pH 7.4) containing 1.0 M NaCl
Flow rate:      1.0 ml/min.
Fraction volume:  1.0 ml/tube/min.

| Concentration gradient: | Time (min) | B% |
|---|---|---|
| | 0 | 0 |
| | 5 | 0 |
| | 55 | 30 |
| | 60 | 100 |
| | 65 | 100 |
| | 70 | 0 |

From the result (elution pattern) of the above DEAE-5PW ion exchange high-performance liquid chromatography, the peaks observed for fractions Nos. 41 and 42 (NaCl concentration 0.21-0.22 M) were found to correspond to the M-CSF. Said peaks were collected to give the purified human M-CSF derivative (E.coli[4-153]-M-CSF) of the invention.

(9) Determination of N-terminal amino acid sequence of M-CSF derivative (E.coli[4-153]-M-CSF)

The N-terminal amino acid sequence of the M-CSF derivative (E.coli[4-153]-M-CSF) obtained by the above procedure (8)-(c-2) was determined using a gaseous phase sequencer (product of Applied Biosystems).

As a result, the sequence Ser-Glu-Tyr-X'-Ser and the sequence Met-Ser-Glu-Tyr-X'-Ser were found in a ratio of about 7-8 to 1.

In the sequences mentioned above, the amino acid X' could not be identified but was estimated to be Cys based on the gene structure.

(10) Improved isolation and purification of M-CSF derivative (E.coli[3-153]-M-CSF)

(a) Preparation of M-CSF fraction from E. coli

To 7.5 g (wet weight) of the E. coli strain SG21058 carrying the plasmid ptrpIL-2X-M-CSF101 was added 1.0 ml of 50 mM Tris-hydrochloride buffer (pH 7.0), and the mixture was stirred thoroughly. Then, 6 ml of 4 mg/ml lysozyme and an amount of EDTA to make a final concentration of 10 mM were added, and the mixture was stirred at 4°C for 15 minutes, then sonicated (20 KHz, 10 minutes, 200 W) and further centrifuged at 10,000 x g/minute for 20 minutes to give a pellet. This was further washed with a buffer for washing (50 mM Tris-hydrochloride containing 2% Triton X-100, pH 7.0) and again subjected to centrifugation under the same conditions. Two repetitions of this procedure gave an M-CSF fraction (pellet).

(b) Refolding of M-CSF from M-CSF fraction

To the M-CSF fraction obtained by the above procedure (a) was added 20 ml of 50 mM Tris-hydrochloride buffer (pH 7.0) containing 7 M guanidine hydrochloride and 25 mM 2-mercaptoethanol, and the mixture was stirred at room temperature for at least 4 hours for solubilization. This solution was gradually added dropwise into a beaker already containing 2,000 ml of 50 mM Tris-hydrochloride buffer (pH 8.5) containing 0.5 mM reduced-form glutathione, 0.1 mM oxidized-form glutathione and 2 M urea (agitated with stirrer). Then, the resultant mixture was allowed to stand at 4°C for at least 2 days. The solution was then centrifuged at 10,000 x g/minute for 30 minutes. The pellet was removed and the supernatant was recovered.

The refolded M-CSF was present in the thus-obtained supernatant.

(c) Purification of M-CSF

The centrifugation supernatant obtained by the above procedure (b) was purified in the following manner.

(c-1) Concentration by ion exchange chromatography

The refolded solution obtained by the above procedure (b) was applied to a QAE-Zeta Prep 100 (product of Pharmacia-LBK) preequilibrated with 50 mM Tris-hydrochloride buffer (pH 8.5) and, after thorough washing with same buffer, the M-CSF fraction was eluted with the above-mentioned buffer containing 0.5 M NaCl.

(c-2) Hydrophobic high-performance liquid chromatography

Ammonium sulfate was added to the fraction obtained in the above manner to give a 30%-saturated solution and the solution was centrifuged at 10,000 x g/minutes for 20 minutes. The pellet was removed and the supernatant was recovered. This supernatant was passed through a 0.45-μm Millipore filter and then subjected to hydrophobic high-performance liquid chromatography under the following conditions.

| | |
|---|---|
| Column: | TSKgel Phenyl 5PW (21.5 mm I.D. x 150 mm, product of Tosoh Corporation) |
| Eluent A: | 30% Ammonium sulfate-saturated 40 mM sodium phosphate buffer (pH 7.4) |
| Eluent B: | 40 mM Sodium phosphate buffer (pH 7.4) |
| Flow rate: | 3.0 ml/min. |
| Fraction volume: | 3.0 ml/tube/min. |

| Concentration gradient: | Time (min) | B% |
|---|---|---|
| | 0 | 0 |
| | 7 | 0 |
| | 47 | 100 |
| | 52 | 100 |
| | 57 | 0 |

As a result, an M-CSF activity was eluted in fractions corresponding to the ammonium sulfate concentrations of 6-3%. Said active fractions were collected and subjected to buffer exchange for 40 mM sodium phosphate (pH 7.4) by ultrafiltration.

(c-3) Anion exchange high-performance liquid chromatography

The fraction obtained by the above procedure (c-2) was subjected to anion exchange high-performance liquid chromatography under the following conditions.

Column:            TSKgel DEAE-5PW (21.5 mm I.D. x 150 mm, product of Tosoh Corporation)
Eluent A:          40 mM Sodium phosphate buffer (pH 7.4)
Eluent B:          40 mM Sodium phosphate buffer (pH 7.4) containing 1.0 M NaCl
Flow rate:         3.0 ml/min.
Fraction volume:   3.0 ml/tube/min.

Concentration gradient:

| Time (min) | B% |
| --- | --- |
| 0 | 0 |
| 5 | 0 |
| 43 | 30 |
| 48 | 100 |
| 53 | 100 |
| 58 | 0 |

From the result (elution pattern) of the above anion exchange high-performance liquid chromatography, the peaks observed with fractions Nos. 35 and 36 (NaCl concentration 0.28-0.29 M) were found to correspond to the M-CSF. Said peaks were collected and thus the purified human M-CSF derivative (E.coli-[3-153]-M-CSF) of the invention was obtained.

⟨Biological activity of human M-CSF derivatives of invention⟩

(1) Blood half-life measurement

The human M-CSF derivative (E.coli[3-214]-M-CSF) obtained in the relevant example and CHO[-32-522]-M-CSF obtained in Reference Example 1 were each diluted with MSA (mouse serum albumin)-containing physiological saline (30 $\mu$g/ml) to a concentration of 20 $\mu$g/ml and these were administered to groups of 4 mice (BALB/c males, 7 weeks of age) intravenously (i.v.) at a dose of 0.25 ml per mouse (200 $\mu$g/kg). After administration, the amount of M-CSF in blood was determined by RIA at timed intervals.

The blood half-life measurement by said RIA was carried out by the following method. Thus, a mixture of 100 $\mu$l of the test sample (blood) or of a standard M-CSF (E.coli[3-153]-M-CSF obtained in Reference Example 2) solution, 100 $\mu$l of a solution containing 10,000 cpm (per 100 $\mu$l) of Na$^{125}$I-labeled M-CSF (derived from the above-mentioned standard M-CSF by labeling with $^{125}$I by the iodogen method) and 200 $\mu$l of an anti-M-CSF rabbit antiserum (prepared by 40,000-fold dilution of the polyclonal antibody OCT511 obtained by the method mentioned later herein with 0.1% BSA/PBS/0.05% thimerosal) is stood at room temperature for 20 hours or at 4°C for 48 hours to thereby allow the antigen-antibody reaction to occur. After completion of the reaction, the labeled M-CSF-bound product and the non-bound product are separated by the two-antibody method. For this separation, 100 $\mu$l of a normal rabbit serum 400-fold diluted with 0.1% BSA/PBS/0.05% thimerosal, 100 $\mu$l of an anti-rabbit IgG serum 40-fold diluted in the same manner and 200 $\mu$l of 12.5% polyethylene glycol solution in PBS are first added, the reaction is allowed to proceed at 4°C for 30 minutes, and centrifugation is conducted at 3,000 rpm for 15 minutes, followed by decantation to give a leveled M-CSF-antibody conjugate as the precipitate. The precipitate is counted with a gamma counter for 1 minute. A standard curve is prepared using standard solutions, the number of counts measured with the test sample containing the M-CSF derivative of the invention is plotted on the standard curve, and the M-CSF concentration in said test sample is determined.

The results obtained are shown below in Table 2.

## Table 2

| Time | M-CSF (I. V. administration) | |
| (min) | CHO[-32-522]-M-CSF | E.coli [3-214]-M-CSF |
|---|---|---|
| 5 | 1335 | 3850 |
| 15 | 891 | 3368 |
| 30 | 623 | 2715 |
| 60 | 434 | 1751 |
| 120 | 272 | 447 |
| 240 | 44 | 124 |
| 1360 | 10 | 19 |
| Half-life | 53.0 min | 46.2 min |

In the table, the unit is ng/ml.

From the above table, it is evident that the half-life of E.coli[3-214]-M-CSF is 46.2 minutes and that of CHO[-32-522]-M-CSF is 53.0 minutes.

The polyclonal antibody against M-CSF as used in the above RIA has the following properties.

1) The polyclonal antibody used in the invention was prepared by the following method.

For polyclonal antibody production, female New Zealand white rabbits weighing 2.5 kg to 3.0 kg were subjected to multipoint intracutaneous immunization with 20 µg/animal of a purified sample of CHO[-32-522]-M-CSF (dissolved in PBS) together with the equal volume of Freund's complete adjuvant, followed by immunizations with 20 µg of the same sample together with Freund's imcomplete adjuvant at one-month intervals. The number of immunizations, inclusive of the first one, amounted to 7. One week after completion of the immunization procedure, the whole blood was collected from the rabbits and antisera were obtained.

One of the three antibodies obtained was designated as OCT511 and stored at -80° C.

2) The polyclonal antibody OCT511 to be used in the determination of blood half-life of each of the derivatives of the invention has the following properties.

[1] Antibody titer

CHO[-32-522]-M-CSF was labeled with $^{125}$I by the iodogen method and the antibody titer was defined as the dilution factor for the antiserum capable of binding 50% of 10 kcpm of this $^{125}$I-labeled CHO[-32-522]-M-CSF.

As a result, the antibody titer of OCT511 was 80,000.

[2] Neutralizing activity

The neutralizing activity was determined by the colony assay method using mouse bone marrow cells. The neutralizing activity of OCT511 was such that 1 ml of OCT511 could neutralize 1 to 2 x $10^6$ units of CHO[-32-522]-M-CSF.

[3] Cross-reactivity

This OCT511 did not cross-react with mouse CSF (L-cell culture supernatant) or with human GM-CSF (Amersham) at all. Furthermore, it did not cross-react at all with human IL-1$\alpha$ (cf. Unexamined Japanese Patent Publication SHO 63-164899), IL-1$\beta$ (cf. Unexamined Japanese Patent Publication SHO 63-152398), IL-2 (Amersham) or TNF-$\alpha$ (Amersham).

24

**Claims**

1. A biologically active recombinant human M-CSF derivative characterized by having a primary amino acid sequence having the C terminus in any position in the region from the 151st position (Thr) to 520th position (Val) in the amino acid sequence defined by the formula shown below, optionally with addition of Met to the N terminus of said sequence or deletion of the N-terminal Val therefrom:

Val-Ser-Glu-Tyr-Cys-Ser-His-Met-Ile-Gly-Ser-Gly-

His-Leu-Gln-Ser-Leu-Gln-Arg-Leu-Ile-Asp-Ser-Gln-

Met-Glu-Thr-Ser-Cys-Gln-Ile-Thr-Phe-Glu-Phe-Val-

Asp-Gln-Glu-Gln-Leu-Lys-Asp-Pro-Val-Cys-Tyr-Leu-

Lys-Lys-Ala-Phe-Leu-Leu-Val-Gln-Asp-Ile-Met-Glu-

Asp-Thr-Met-Arg-Phe-Arg-Asp-Asn-Thr-Pro-Asn-Ala-

Ile-Ala-Ile-Val-Gln-Leu-Gln-Glu-Leu-Ser-Leu-Arg-

Leu-Lys-Ser-Cys-Phe-Thr-Lys-Asp-Tyr-Glu-Glu-His-

Asp-Lys-Ala-Cys-Val-Arg-Thr-Phe-Tyr-Glu-Thr-Pro-

Leu-Gln-Leu-Leu-Glu-Lys-Val-Lys-Asn-Val-Phe-Asn-

Glu-Thr-Lys-Asn-Leu-Leu-Asp-Lys-Asp-Trp-Asn-Ile-

Phe-Ser-Lys-Asn-Cys-Asn-Asn-Ser-Phe-Ala-Glu-Cys-

Ser-Ser-Gln-Asp-Val-Val-Thr-Lys-Pro-Asp-Cys-Asn-

Cys-Leu-Tyr-Pro-Lys-Ala-Ile-Pro-Ser-Ser-Asp-Pro-

Ala-Ser-Val-Ser-Pro-His-Gln-Pro-Leu-Ala-Pro-Ser-

Met-Ala-Pro-Val-Ala-Gly-Leu-Thr-Trp-Glu-Asp-Ser-

Glu-Gly-Thr-Glu-Gly-Ser-Ser-Leu-Leu-Pro-Gly-Glu-
Gln-Pro-Leu-His-Thr-Val-Asp-Pro-Gly-Ser-Ala-Lys-
Gln-Arg-Pro-Pro-Arg-Ser-Thr-Cys-Gln-Ser-Phe-Glu-
Pro-Pro-Glu-Thr-Pro-Val-Val-Lys-Asp-Ser-Thr-Ile-
Gly-Gly-Ser-Pro-Gln-Pro-Arg-Pro-Ser-Val-Gly-Ala-
Phe-Asn-Pro-Gly-Met-Glu-Asp-Ile-Leu-Asp-Ser-Ala-
Met-Gly-Thr-Asn-Trp-Val-Pro-Glu-Glu-Ala-Ser-Gly-
Glu-Ala-Ser-Glu-Ile-Pro-Val-Pro-Gln-Gly-Thr-Glu-
Leu-Ser-Pro-Ser-Arg-Pro-Gly-Gly-Gly-Ser-Met-Gln-
Thr-Glu-Pro-Ala-Arg-Pro-Ser-Asn-Phe-Leu-Ser-Ala-
Ser-Ser-Pro-Leu-Pro-Ala-Ser-Ala-Lys-Gly-Gln-Gln-
Pro-Ala-Asp-Val-Thr-Gly-Thr-Ala-Leu-Pro-Arg-Val-
Gly-Pro-Val-Arg-Pro-Thr-Gly-Gln-Asp-Trp-Asn-His-
Thr-Pro-Gln-Lys-Thr-Asp-His-Pro-ser-Ala-Leu-Leu-
Arg-Asp-Pro-Pro-Glu-Pro-Gly-Ser-Pro-Arg-Ile-Ser-
Ser-Pro-Arg-Pro-Gln-Gly-Leu-Ser-Asn-Pro-Ser-Thr-
Leu-Ser-Ala-Gln-Pro-Gln-Leu-Ser-Arg-Ser-His-Ser-
Ser-Gly-Ser-Val-Leu-Pro-Leu-Gly-Glu-Leu-Glu-Gly-
Arg-Arg-Ser-Thr-Arg-Asp-Arg-Arg-Ser-Pro-Ala-Glu-
Pro-Glu-Gly-Gly-Pro-Ala-Ser-Glu-Gly-Ala-Ala-Arg-
Pro-Leu-Pro-Arg-Phe-Asn-Ser-Val-Pro-Leu-Thr-Asp-
Thr-Gly-His-Glu-Arg-Gln-Ser-Glu-Gly-Ser-Ser-Ser-
Pro-Gln-Leu-Gln-Glu-Ser-Val-Phe-His-Leu-Leu-Val-
Pro-Ser-Val-Ile-Leu-Val-Leu-Leu-Ala-Val-Gly-Gly-

```
Leu-Leu-Phe-Tyr-Arg-Trp-Arg-Arg-Arg-Ser-His-Gln-

Glu-Pro-Gln-Arg-Ala-Asp-Ser-Pro-Leu-Glu-Gln-Pro-

Glu-Gly-Ser-Pro-Leu-Thr-Gln-Asp-Asp-Arg-Gln-Val-

Glu-Leu-Pro-Val
```

2. An M-CSF derivative as defined in Claim 1 which has the primary amino acid sequence from the 1st position Val to the 182nd position Ala.

3. An M-CSF derivative as defined in Claim 1 which has the primary amino acid sequence from the 1st position Val to the 212nd position Pro.

4. An M-CSF derivative as defined in Claim 1 which has the primary amino acid sequence from the 1st position Val to the 256th position Gly.

5. An M-CSF derivative as defined in Claim 1 which has the primary amino acid sequence from the 1st position Val to the 300th position Gln.

6. An M-CSF derivative as defined in Claim 1 which has the primary amino acid sequence from the 1st position Val to the 332nd position Ala.

7. An M-CSF derivative as defined in Claim 1 which has the primary amino acid sequence from the 1st position Val to the 520th position Val.

8. An M-CSF derivative as defined in Claim 1 which has the primary amino acid sequence from the 2nd position Ser to the 151st position Thr.

9. An M-CSF derivative as defined in Claim 1 which has the primary amino acid sequence from the 2nd position Ser to the 182nd position Ala.

10. An M-CSF derivative as defined in Claim 1 which has the primary amino acid sequence from the 2nd position Ser to the 212nd position Pro.

11. An M-CSF derivative as defined in Claim 1 which has the primary amino acid sequence from the 2nd position Ser to the 256th position Gly.

12. An M-CSF derivative as defined in Claim 1 which has the primary amino acid sequence from the 2nd position Ser to the 300th position Gln.

13. An M-CSF derivative as defined in Claim 1 which has the primary amino acid sequence from the 2nd position Ser to the 332nd position Ala.

14. An expression vector for any of the M-CSF derivatives defined in Claims 2-13.

15. A vector as defined in Claim 14 which is ptrpIL-2X-M-CSF201, ptrpIL-2X-M-CSF202, ptrpIL-2X-M-CSF203, ptrpIL-2X-M-CSF204, ptrpIL-2X-M-CSF205, ptrpIL-2X-M-CSF206, ptrpIL-2X-M-CSF109, ptrpIL-2X-M-CSF402, ptrpIL-2X-M-CSF403, ptrpIL-2X-M-CSF404, ptrpIL-2X-M-CSF405 or ptrpIL-2X-M-CSF406.

16. A transformant as transformed with any of the vectors defined in Claim 14.

17. A transformant as defined in Claim 16 which is E. coli SG21058/ptrpIL-2X-M-CSF201, E. coli SG21058/ptrpIL-2X-M-CSF202, E. coli SG21058/ptrpIL-2X-M-CSF203, E. coli SG21058/ptrpIL-2X-M-

27

CSF204, E. coli SG21058/ptrpIL-2X-M-CSF205, E. coli SG21058/ptrpIL-2X-M-CSF206, E. coli SG21058/ptrpIL-2X-M-CSF109, E. coli SG21058/ptrpIL-2X-M-CSF402, E. coli SG21058/ptrpIL-2X-M-CSF403, E. coli SG21058/ptrpIL-2X-M-CSF404, E. coli SG21058/ptrpIL-2X-M-CSF405 or E. coli SG21058/ptrpIL-2X-M-CSF406.

18. A method of producing the M-CSF derivatives as defined in Claim 1 which comprises cultivating the transformant defined in Claim 16 and recovering the M-CSF expressed thereby.

# INTERNATIONAL SEARCH REPORT

**I. CLASSIFICATION OF SUBJECT MATTER** (if several classification symbols apply, indicate all) 6

According to International Patent Classification (IPC) or to both National Classification and IPC    Int. $Cl^5$

C07K13/00, C12N15/27, 1/21, C12P21/02//A61K37/02
(C12N1/21, C12R1:19)(C12P21/02, C12R1:19)

**II. FIELDS SEARCHED**

| Minimum Documentation Searched 7 | |
|---|---|
| Classification System | Classification Symbols |
| IPC | C07K13/00, C12N1/21, 15/27, C12P21/02, A61K37/02 |

Documentation Searched other than Minimum Documentation
to the Extent that such Documents are Included in the Fields Searched 8

Biological Abstracts Data Base (BIOSIS)

**III. DOCUMENTS CONSIDERED TO BE RELEVANT** 9

| Category * | Citation of Document, 11 with indication, where appropriate, of the relevant passages 12 | Relevant to Claim No. 13 |
|---|---|---|
| A | JP, A, 1-104176 (Otsuka Pharmaceutical Co., Ltd.), April 21, 1989 (21. 04. 89), & EP, A1, 261592 | 1-18 |
| A | JP, A, 1-501283 (Shitas Corp.), May 11, 1989 (11. 05. 89), & WO, A1, 88/3173 & EP, A2, 272779 | 1-18 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 25, 1991 (25. 01. 91) | February 12, 1991 (12. 02. 91) |
| International Searching Authority | Signature of Authorized Officer |
| Japanese Patent Office | |